# EUROPEAN PATENT APPLICATION

(11) **EP 4 534 001 A1**
(43) Date of publication of application: **09.04.2025**
(21) Application number: 24202151.7
(22) Date of filing: 24.09.2024
(51) Int. Cl.: A61B 5/00, A61B 5/01, A61B 5/0205, A61B 5/024, A61B 5/11, A61B 5/1455

(54) **ELECTRONIC DEVICE AND RELATED METHODS FOR DATA COLLECTION**

(30) Priority: 05.10.2023 SE 2351150
(71) Applicant: Sony Network Communications Europe B.V., 2132LS Hoofddorp (NL)
(72) Inventor: TENNHAMMAR, Jessica, Basingstoke, RG22 4SB (GB); ROCKLIND, Johan, Basingstoke, RG22 4SB (GB)
(74) Representative: Aera A/S

(57) **Abstract**

A wearable device is disclosed. The wearable device comprises memory circuitry, processor circuitry, an interface. The wearable device is configured to obtain physiological data associated with location data. The location data is indicative of a location of the wearable device. The location data has the same timestamp as the physiological data. The wearable device is configured to provide the physiological data associated with the location data.

## Description

### TECHNICAL FIELD

The present disclosure pertains to the field of Internet-of-Things. The present disclosure relates to electronic devices and related methods for data collection.

### BACKGROUND

Scientific studies, such as clinical studies, may investigate and evaluate the safety and efficacy of a medical intervention, such as a new drug, medical device, vaccine, treatment, etc. Clinical trials can constitute crucial steps forward in the process of developing and/or accessing new medical interventions prior to approval for widespread treatment.

Obtaining accurate data for use in a scientific context, such as clinical studies, may be a complex task requiring diligence in various aspects to account for as many variables as possible.

It may especially be difficult to ensure the accuracy of the data obtained as it is often provided by the patients themselves, who may provide inaccurate data. This introduces uncertainty into the obtained data and may make it difficult to draw accurate and principled scientific conclusions based on such data.

### SUMMARY

Accordingly, there is a need for devices and methods for data collection, which may mitigate, alleviate, or address the shortcomings existing and may allow for a more accurate and robust collection of data, such as physiological data associated with a location.

A wearable device is disclosed. The wearable device comprises memory circuitry, processor circuitry, and an interface. The wearable device is configured to obtain physiological data associated with location data. The location data is indicative of a location of the wearable device. The location data has the same timestamp as the physiological data. The wearable device is configured to provide the physiological data associated with the location data.

A method, performed by a wearable device, is disclosed. The method comprises obtaining physiological data associated with location data. The location data is indicative of a location of the wearable device. The location data has the same timestamp as the physiological data. The method comprises providing the physiological data associated with the location data.

An electronic device is disclosed. The electronic device comprises memory circuitry, processor circuitry, and an interface. The electronic device is configured to receive, from a wearable device, physiological data. The physiological data is associated with location data indicative of a location of the wearable device. The location data has the same timestamp as the physiological data.

A method, performed by an electronic device, is disclosed. The method comprises receiving, from a wearable device, physiological data. The physiological data is associated with location data indicative of a location of the wearable device. The location data has the same timestamp as the physiological data.

It is an advantage of the present disclosure that the physiological data associated with a user, such as a user of the wearable device, may be provided with a corresponding location and timestamp. Advantageously, the timestamp may be associated using the wearable device, thereby improving the accuracy of the correspondence between the obtained data and the corresponding location and timestamp (such as by eliminating time lag potentially caused by the wearable device obtaining the location and/or timestamp externally, such as from an external server device, which may cause the timestamp and/or location corresponding to the raw physiological data to be inaccurate). In other words, the location data and/or timestamp obtained by the wearable device enable an improved accuracy as it is obtained in the same wearable device (such as hardware) as the obtained physiological data associated with the wearer (such as participant of clinical trial). This may advantageously allow for improved quality and/or accuracy of the raw physiological data used for clinical trials, thereby improving the overall quality of the results and/or conclusions derived based on the clinical data.

Furthermore, providing the physiological data with a location and timestamp allows for assessing various factors related to the location and time of collection which may affect the physiological data. This allows for more robustness of the physiological data thanks to cross-referencing, correlating, and compiling of the physiological data with other (such as environmental) data sets.

Further, the location and/or timestamp corresponding with data points of the physiological data set may be used to find a correspondence or comparison to data points of one or more different data sets, such as data sets obtained by third party studies, institutions, individuals, etc. This may advantageously enable improved accuracy and quantifiability of the effect that variables/factors may have had on the physiological data of the wearer of the wearable device during a given study, thereby improving the reliability of the results in various applications, such as clinical trials.

### BRIEF DESCRIPTION OF THE DRAWINGS

The above and other features and advantages of the present disclosure will become readily apparent to those skilled in the art by the following detailed description of examples thereof with reference to the attached drawings, in which:
Fig. 1 is a diagram illustrating an example system according to this disclosure,
Fig. 2 is a flow-chart illustrating an example method, performed in a wearable device for data collection, according to this disclosure, and
Fig. 3 is a flow-chart illustrating an example method, performed in an electronic device for data collection, according to this disclosure.

### DETAILED DESCRIPTION

Various examples and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the examples. They are not intended as an exhaustive description of the disclosure or as a limitation on the scope of the disclosure. In addition, an illustrated example needs not have all the aspects or advantages shown. An aspect or an advantage described in conjunction with a particular example is not necessarily limited to that example and can be practiced in any other examples even if not so illustrated, or if not so explicitly described.

The figures are schematic and simplified for clarity, and they merely show details which aid understanding the disclosure, while other details have been left out. Throughout, the same reference numerals are used for identical or corresponding parts.

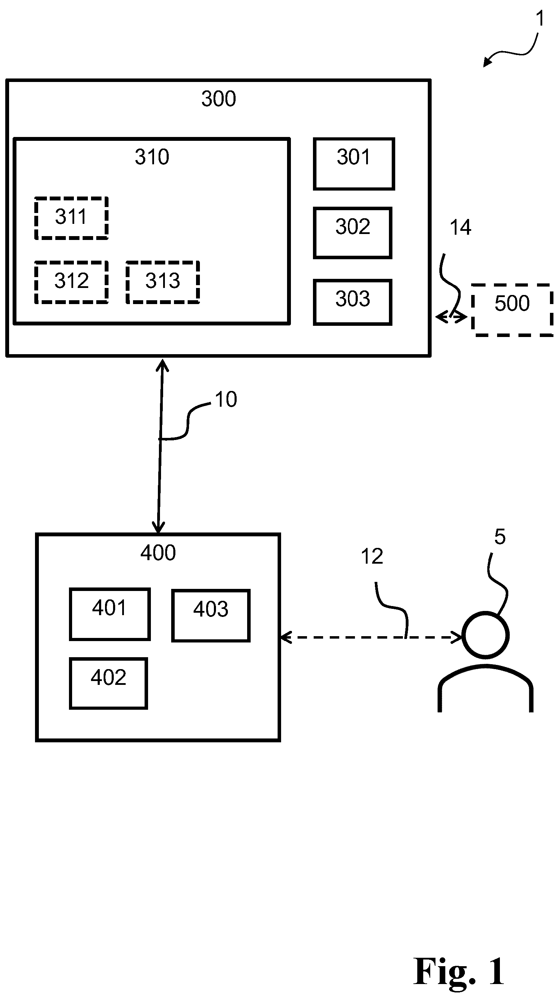

A wearable device disclosed herein can be seen as a device worn by a user. For example, the wearable device may be seen as a worn electronic device. In some examples, the wearable device may be a wrist-worn device, such as a smart watch. In some examples, the wearable device may be a device integrated in clothes worn by the user. A wearer can be seen as a person wearing the wearable device, such as the wearable device 300 of Fig. 1.

Physiological data can be seen as data associated with a physiological state/condition of a user of the wearable device disclosed herein. For example, the physiological data can be obtained by monitoring one or more vitals of the user, and/or one or more biomarkers of the user. For example, the physiological data can be seen as one or more of: health data, biological data, physical data, and clinical data. The physiological data according to this disclosure is not preprocessed (for example, manipulated, transformed, and/or filtered, etc.) before transmission. In other words, the physiological data can be seen as raw physiological data.

Physical activity data can be seen as data indicative of a physical activity of the user of the wearable device including movement and/or sleep. For example, the physical activity data can include: physical measurement data, performance outcome measurements, functional performance measurements, and/or movement measurements. In one or more example wearable devices, the physical activity data comprises movement data and/or sleep data.

The present disclosure provides, inter alia, a digital biomarker solution for remote collection of physiological data (such as raw physiological data) and/or physical activity data that can be used in clinical studies.

The disclosed solution(s) can be used for example by pharma companies, contract research organisations (CRO) and digital biomarker companies for studies measuring functional measures.

The disclosed solution(s) can be seen as a system to control data collection in clinical trials and/or studies by utilizing remote collection of raw data from various sensors (such as accelerometer, gyroscope, and/or photoplethysmogram sensor) associated with the wearable device.

For example, participants in a clinical study are provided with a wearable device disclosed herein (such as a wrist worn device) that collects physiological and/or physical activity data to be used in the clinical study. For example, the clinical study can be a clinical study that uses functional measures in the investigations and/or a clinical study that uses evaluations of the safety and efficacy of a medical intervention.

The disclosed wearable device can be used for clinical studies of conditions such as: Neurodegenerative Disorders (such as Alzheimer's, Parkinson, and/or epilepsy), sleep and movement diseases (such as insomnia, sleep apnea, and/or parasomnia) and/or possibly cardiovascular condition, oncology, pain, rheumatism etc.

The present disclosure proposes, inter alia, adding geographic location as a complement to the physiological data collection and/or physical activity data collection.

Participants reporting the locations they have been at does not give as accurate position as collecting it via satellites or network nodes with timestamps aligned with the rest of the collected data.

Location data obtained via the wearable device disclosed herein enables the possibility of adding complementing external data to the study analysis. The external data can for example include pollen data, climate and weather conditions, pollution data, sun radiation (UV) data, geographic locations that can be classified as stressful etc.

Fig. 1 is a diagram illustrating an example system 1 comprising an example electronic device 400 and an example wearable device 300 according to this disclosure.

The system 1 described herein comprises a wearable device 300. The wearable device 300 comprises memory circuitry 301, processor circuitry 302, and an interface 303. The interface 303 for example comprises a wireless interface, such as for short-range and /or cellular communications. In other words, the wearable device 300 may be configured for data collection. In some examples, the wearable device 300 is a data collection wearable device. In some examples, the wearable device 300 is a timestamping wearable device.

In some examples, the system 1 comprises an electronic device 400. The electronic device 400 can for example be seen as an external electronic device, such as external to the wearable device 300. The electronic device 400 is for example a laptop or desktop computer, and/or a server device in a cloud architecture. The electronic device 400 is for example configured to run a front-end program and to be controlled by a server device (such as a backend server device), such as via communication. In some examples, the electronic device 400 is part of a platform for monitoring, configuring, and/or managing wearables for clinical studies.

The wearable device 300 may be configured to communicate with the electronic device 400 via a wireless link (or radio access link) 10. For example, the electronic device 400 is configured to receive, from the wearable device 300, physiological data, location data, and/or physical activity data. The physiological data and/or physical activity data received by the electronic device 400, from the wearable device 300, is for example associated with location data indicative of the location of the wearable device 300. The location data for example has the same timestamp as the physiological data and/or the physical activity data.

Optionally, when the electronic device 400 is for example laptop or desktop computer, the electronic device 400 is for example configured to receive a user input 12 provided by the user 5. The user 5 may for example be a clinician, such as a clinician associated with a clinical trial. The user input 12 can be provided directly or indirectly (such as via one or more devices of a platform) to the electronic device 400.

For example, the user input 12 is for configuration of the wearable device 300. For example, the electronic device 400 may be configured to set up, based on the user input 12, one or more configuration parameters. The electronic device 400 may be configured to provide, such as via wireless link 10, the one or more configuration parameters to the wearable device 300. The wearable device 300 may for example be configured to operate according to one or more configuration parameters. In some examples, the electronic device 400 is for example configured to operate according to a user input 12, provided by a user 5.

The wearable device 300 is configured to obtain (such as via the one or more sensors 310) physiological data associated with location data. The location data is indicative of a location of the wearable device. The location data has the same timestamp as the physiological data.

Physiological data can be seen as data associated with a physiological state and/or a physiological condition of a wearer, such as a wearer of the wearable device 300. The physiological data for example comprises one or more of: health data, biological data, physical data, clinical data. The physiological data for example comprises raw (such as un-processed, un-altered, un-modified, etc) data. In other words, the physiological data can be seen as the raw data obtained via a physiological sensor.

The location data for example comprises information indicative of the location of the wearable device 300, such as geographic location, such as coordinates. For example, the location data for example comprises one or more values (such as coordinates) indicative of the location of the wearable device 300.

The timestamp is for example indicative of a time and/or date. For example, the timestamp can for example be seen as a record of a time and/or date, for example provided by an internal clock of the wearable device 300. The timestamp for example comprises one or more values indicative of a time and/or date.

The wearable device 300 is configured to provide (such as via the interface 303) the physiological data associated with the location data. In one or more example wearable devices, the wearable device 300 is configured to transmit the physiological data associated with the location data and/or the physical activity data associated with the location data, to an external electronic device, such as electronic device 400. For example, the wearable device 300 sends (such as via the interface 303) the physiological data associated with the location data, to the electronic device 400, for example for further processing.

The external electronic device can for example be seen as an electronic device different to the wearable device 300. In some examples, the electronic device 400 is an external electronic device. The external electronic device may for example be an electronic device of a clinician, such as an electronic device configured to be operated by a clinician. For example, the user 5 may be a clinician.

In some examples, the external electronic device is a cloud device, such as a server device. In some examples, the external electronic device is configured to run a clinical trial platform.

In some examples, the wearable device 300 may be configured to transmit physiological data associated with the location data and/or the physical activity data associated with the location data, to an external electronic device using wireless communication, such as short-range communication (such as Near Field Communication (NFC), such as Bluetooth Low Energy (BLE)) and/or cellular communication.

In one or more example wearable devices, the physiological data comprises one or more of: photoplethysmogram (PPG) data, temperature data, and electrocardiogram data. The physiological sensor may for example comprise a PPG sensor, a thermal sensor and/or an ECG sensor. The physiological sensor may comprise other suitable sensor(s). The PPG data is for example obtained using hardware of the wearable device 300. For example, the PPG data may be obtained using a physiological sensor, such as a PPG sensor. For example, PPG data may be obtained by a light sensor such as a photodetector. The temperature data and/or the electrocardiogram (ECG) data may for example be obtained from an external sensor 500 wirelessly, such as via link 14, for example, using Near Field Communication, such as Bluetooth Low Energy (BLE) communication. The PPG data for example comprises one or more of: heart rate data, heart rate variability data and respiratory data. Heart rate data, for example, comprises information indicative of a heart rate. The heart rate data is for example based on the PPG data obtained by the wearable device 300. The heart rate data is for example determined, such as based on the PPG data, by the wearable device 300 and/or by an electronic device (such as the electronic device 400). For example, the heart rate data comprises a beat per minute (bpm) of a heart. Heart rate variability data is for example associated with the bpm of a heart. For example, the heart rate variability may be indicative of the variation of the bpm, such as over a given time period.

Respiratory data is for example associated with the respiration of a wearer of the wearable device 300. For example, the respiratory data may be associated with the state, activity and/or condition of lungs of the wearer. The respiratory data may for example be obtained via a physiological sensor, such as a respiratory sensor, a stretch sensitivity sensor (for example worn on a torso) and/or a motion sensor (such as a gyroscope and/or an accelerometer). In some examples, the respiratory rate can be determined based on data from the motion sensor.

Temperature data comprises information indicative of a temperature, such as a skin temperature of the wearer. Temperature data may be obtained by a sensor of the one or more sensors, such as a thermal sensor.

Electrocardiogram data for example comprises information indicative of the electrical activity of the heart of the user of the wearable device 300. The electrocardiogram data may for example be indicative of the state, activity and/or condition of the heart, such as of the wearer. Electrocardiogram data may for example be obtained using a sensor of the one or more sensors, such as an electrocardiogram sensor, such as an electrode. In some examples, the physiological data may comprise stress data, such as associated with the stress state and/or stress condition of a wearer of the wearable device 300.

In one or more example wearable devices, the wearable device 300 is configured to obtain the physiological data by obtaining the physiological data and the location data from one or more sensors. For example, the physiological data can be seen as raw physiological data, not yet timestamped by the internal clock of the wearable device 200. In one or more example devices, the one or more sensors comprise one or more sensors internal to the wearable device 300 and/or one or more sensors external to the wearable device 300.

In the example of Fig. 1, the wearable device 300 comprises one or more sensors 310. In one or more example wearable devices, the one or more sensors 310 comprise a location sensor 311, a physiological sensor 312, and/or a physical activity sensor 313. In some examples, the one or more sensors 310 of the wearable device 300 comprise the location sensor 311, the physiological sensor 312, and the physical activity sensor 313. The location sensor 311 is, for example, configured to obtain location data. The physiological sensor 312 is, for example, configured to obtain physiological data. The physical activity sensor 313 is, for example, configured to obtain physical activity data.

In some examples, obtaining the physiological data comprises retrieving and/or receiving the physiological data from the one or more sensors. In some examples, obtaining the physiological data comprises generating and/or determining the physiological data.

The wearable device 300 is, for example, configured to obtain location data using the location sensor 311. In some examples, the location sensor 311 is a part of (such as internal to) the wearable device 300. The wearable device 300 is, for example,, configured to obtain physiological data using the physiological sensor 312. The wearable device is configured to obtain physical activity data using the physical activity sensor 313.

In one or more example wearable devices, the one or more sensors internal to the wearable device 300 can be seen as being located (at least partially) inside of an external surface (such as housing, casing and/or shell) of the wearable device. For example, the one or more sensors internal to the wearable device 300 may not be visible to a wearer of the wearable device 300 (such as when the external surface of the wearable device is opaque). In some examples, the one or more sensors internal to the wearable device 300 may be partially visible to a wearer of the wearable device 300.

In one or more example wearable devices, the one or more sensors external to the wearable device 300 may be associated with an electronic device different to the wearable device. For example, the one or more sensors external to the wearable device 300 may be associated with (such as a part of) positioning equipment (such as a positioning beacon). In some examples, the one or more sensors external to the wearable device 300 may be associated with equipment and/or machinery associated with health monitoring of a wearer, such as hospital equipment and/or hospital machinery.

In one or more example wearable devices, the wearable device 300 is configured to obtain (such as receive and/or retrieve) the physiological data by generating a timestamp parameter for the physiological data and the location data using the same electronic clock. In other words, for example, the wearable device 300 receives and/or retrieves the physiological data (such as raw physiological data) and generates a timestamp parameter for the physiological data and the location data using the same electronic clock. The timestamp parameter can be seen as a parameter providing a timestamp indicating the time and date for the physiological data and the location data. In other words, the timestamp parameter may comprise one or more values indicative of the time and date. Stated differently, for example, the wearable device 300 timestamps the physiological data and the location data using the same electronic clock.

The electronic clock can for example be seen as a time-keeping device. The electronic clock is for example a software driven clock. In some examples, the electronic clock is a real time clock (RTC). In one or more example wearable devices, the electronic clock is time synchronised via a network, such as Network Time Protocol (NTP).

In some examples, the wearable device 300 comprises the electronic clock. For example, the electronic clock is a microcontroller unit (MCU) of the processing circuitry 302. In some examples, a motherboard of the electronic device can be configured to operate as an electronic clock.

The timestamp parameter is for example based on the electronic clock. In some examples, the timestamp parameter may be provided by the electronic clock. In some examples, the wearable device 300 is configured to obtain (such as receive and/or retrieve) the same timestamp (such as from the same electronic clock) for the physiological data and the location data.

In one or more example wearable devices, the wearable device 300 is configured to obtain the physiological data by associating the timestamp parameter with the physiological data and the location data. In some examples, associating the timestamp parameter with the physiological data and the location data can be seen as labelling, tagging and/or marking the physiological data and the location data with the timestamp parameter. For example, the wearable device 300 tags, labels and/or associates the generated timestamp parameter with the physiological data and the location data.

In some examples, when the one or more sensors are external to the wearable device 300, an external electronic device associated with the one or more sensors external to the wearable device 300 may be configured to associate the timestamp parameter with the obtained data (such as physiological data and/or physical activity data). In some examples, the external device may have a clock that is synchronized with the clock of the wearable device. In some examples, the external sensor(s) may have a clock that is synchronized with the clock of the wearable device. Associating the timestamp parameter and the obtained data at the external electronic device where the data is obtained may enable improved accuracy of the timestamp (such as by reducing time between data obtained and timestamp association, such as caused by transmission of data to device which obtained data to the wearable device).

In one or more example wearable devices, the wearable device 300 is configured to obtain (such as via the interface 303 and/or processor circuitry 301) physical activity data associated with the location data. In one or more example wearable devices, the physical activity data is indicative of a physical activity including movement and/or sleep. In one or more example wearable devices, the location data has the same timestamp as the physical activity data.

In one or more example wearable devices, the physical activity data comprises movement data and/or sleep data. In one or more example embodiments, physical activity data can be seen as physical measurement data, performance outcome measurements, functional performance measurements, and/or movement measurements. In some examples, the physical activity data comprises processed data. For example, the movement data can be obtained from a motion sensor. For example, the sleep data can be obtained from a sleep sensor. For example, sleep data can be calculated based on raw data. For example, processed can be provided, such as in fall detection.

In one or more example wearable devices, the wearable device 300 is configured to provide (such as via the interface 303) the physical activity data associated with the location data. In some examples, the wearable device 300 is configured to obtain (such as receive and/or retrieve) the same timestamp (such as from the same electronic clock) for the physical activity data and the location data and provide the physical activity data and the location data having the same timestamp. For example, the same timestamp is provided for the physical data and the location data by having the same timestamping source providing the timestamp, such as the same electronic clock.

In one or more example wearable devices, the movement data comprises accelerometer data and/or gyroscope data. The physical activity sensor may for example be an accelerometer and/or a gyroscope. Accelerometer data can for example be seen as data indicative of an acceleration of the wearable device 300. The wearable device 300 is, for example, configured to obtain accelerometer data using an accelerometer. The accelerometer is for example internal or external to the wearable device 300. Gyroscope data can for example be seen as data indicative of the rotational motion of the wearable device 300. The wearable device is, for example, configured to obtain gyroscope data using a gyroscope. The gyroscope is for example internal or external to the wearable device. In some examples, the sleep data can be determined based on the movement data and/or the physiological data (such as the PPG data).

In one or more example wearable devices, the interface comprises a wireless interface. The interface (such as the interface 303 shown in Fig. 1) can include an interface configured for wireless communication. For example, the wearable device may be configured to communicate wirelessly using a wireless interface. In some examples, the wireless interface is a silicon integrated circuit chip. For example, the wireless interface may be a Subscriber Identity Module (SIM) card.

In some examples, the wearable device 300 may be configured to communicate the physiological data using the wireless interface. The wireless interface may enable the wearable device 300 to directly provide (such as transmit) the physiological data, location data and/or the physical activity data, such as to an electronic device, such as the electronic device 400, for timely control of the wearable device 300. This may advantageously reduce the need for prompting users to provide the physiological data and/or physical activity data. Further, directly (for example, in real time) providing (such as fetching, pulling) the physiological data, location data and/or the physical activity data may improve the accuracy and/or quality of the provided data.

In one or more example wearable devices, the wireless interface may be a digital wireless interface. For example, the wireless interface may be an embedded Subscriber Identity Module (eSIM) card. The wireless interface can be used to generate the location data.

In one or more example wearable devices, the location data comprises one or more of: satellite navigation data, cellular positioning data, and beacon positioning data. Satellite navigation data, for example, comprises navigation data obtained using satellite communication. For example, the satellite navigation data may be indicative of the location of the wearable device. The satellite navigation data may be seen as satellite positioning data. The satellite navigation data for example comprises Global Positioning System (GPS) data, Globalnaya Navigazionnaya Sputnikovaya Sistema (GLONASS) data, Beidou data and/or Galileo data. Cellular positioning data, for example, comprises positioning data obtained using cellular communication, such as between the wearable device 300 and a network node (such as a base station). For example, the cellular positioning data may be indicative of the location of the wearable device 300. Beacon positioning data, for example, comprises positioning data obtained using one or more beacons, such as positioning beacons. For example, the wearable device 300 may be configured to communicate with one or more beacons to obtain the beacon positioning data.

The beacon positioning data is for example indicative of the location of the wearable device 300.

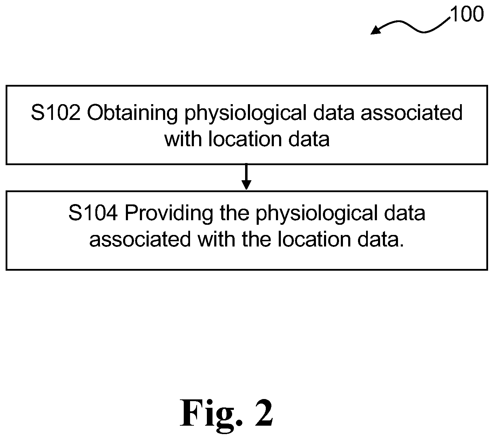

The wearable device 300 is optionally configured to perform any of the operations disclosed in Fig. 2 (such as any one or more of S102, S104). The operations of the wearable device 300 may be embodied in the form of executable logic routines (for example, lines of code, software programs, etc.) that are stored on a non-transitory computer readable medium (for example, memory circuitry 301) and are executed by processor circuitry 302.

Furthermore, the operations of the wearable device 300 may be considered a method that the wearable device 300 is configured to carry out. Also, while the described functions and operations may be implemented in software, such functionality may also be carried out via dedicated hardware or firmware, or some combination of hardware, firmware and/or software. Memory circuitry 301 may be one or more of a buffer, a flash memory, a hard drive, a removable media, a volatile memory, a non-volatile memory, a random access memory (RAM), or other suitable device. In a typical arrangement, memory circuitry 301 may include a non-volatile memory for long term data storage and a volatile memory that functions as system memory for processor circuitry 302. Memory circuitry 301 may exchange data with processor circuitry 302 over a data bus. Control lines and an address bus between memory circuitry 301 and processor circuitry 302 also may be present (not shown in Fig. 1). Memory circuitry 301 is considered a non-transitory computer readable medium.

Memory circuitry 301 may be configured to store physiological data, location data, physical activity data, and/or a timestamp parameter in a part of the memory.

Fig. 1 shows an example electronic device 400 disclosed herein. The electronic device 400 comprises memory circuitry 401, processor circuitry 402, and an interface 403. The electronic device 400 is configured to receive (such as via the interface 403), from a wearable device, physiological data. The physiological data is associated with location data indicative of a location of the wearable device 300. The location data has the same timestamp as the physiological data. The electronic device 400 may be seen as a data sink device, that collects and processes the data received from the wearable device 300 being the data source device. Stated differently, the electronic device 400 is for example coupled with the wearable device 300 for monitoring vitals of a patient.

In other words, the electronic device 400 may be configured for data collection. In some examples, the electronic device 400 is a compiling electronic device. In some examples, the electronic device is a data compiler electronic device.

The electronic device 400 is for example communicatively coupled with the wearable device 300 for monitoring vitals of a patient. The electronic device 400 can for example be seen as the sink for the received physiological data. The electronic device 400 may for example be configured to correlate and/or to perform further processing the physiological data.

The electronic device 400 is, for example, a user device (such as smart phone, and/or laptop), and/or a server device.

In one or more example electronic devices, the electronic device 400 is configured to receive (such as via the interface 403), physical activity data. The physical activity data is associated with location data indicative of a location of the wearable device. The location data has the same timestamp as the physical activity data.

In one or more example electronic devices, the electronic device 400 is configured to receive (such as via the interface 403) user input 12 for configuration of the wearable device.

In one or more example electronic devices, the electronic device 400 is configured to set up one or more configuration parameters associated with the physiological data, location data and/or physical activity data according to the user input, for example, before, during and/or after the monitoring by the wearable device 300. The user input 12 can for example be seen as a user input from a user 5, such as a clinician. For example, the configuration parameter(s) can provide a configuration of a type of the physiological data, location data and/or physical activity data to be monitored by the wearable device 300.

In one or more examples, the configuration parameters comprise one or more of: sample rate of a sensors, additional data to be retrieved (such as environment data indicative of conditions at the location and time of the physiological data and/or physical activity data), the type of the physiological data (such as photoplethysmogram data, temperature data, and/or electrocardiogram data) and/or the type of physical activity data (such as movement data and/or sleep data) and/or the type of location data, and location parameters (such as periodicity of the location data, such as every 1h, per 1 ms, per 1s). For example, the user may provide a user input 12 for selecting the physiological data to be temperature data, and electrocardiogram data and the physical data to be movement data.

In one or more example electronic devices, the electronic device 400 is configured to obtain (such as interface 403, and/or processing circuitry 402), based on the physiological data associated with the location data and/or the physical activity data, environment data indicative of conditions at the location and time of the physiological data.

The electronic device 400 is for example configured to obtain (such as receive and/or retrieve) the environment data. In some examples, the electronic device 400 is configured to obtain the environment data from a platform, and/or a local device. The electronic device 400 may be configured to obtain the environment from one or more service providers and/or applications. The electronic device 400 may be configured to obtain the environment data from a database and/or a data store. The environment data can for example be seen as environmental conditions. In other words, the environment data comprises information associated with the surroundings of the wearer of the wearable device 300. For example, the environment data may be indicative of meteorological conditions and/or atmospheric conditions.

In one or more example electronic devices, the environment data comprises one or more of: weather data, pollution data, pollen data, and sun radiation data. Weather data may comprise information indicative of the weather, for example at a given location corresponding to the location data, such as rainfall, temperature, humidity, wind speed, etc.

Pollution data may comprise information indicative of pollution. Pollution data for example comprises light pollution data, air pollution data, water pollution data, etc. In some examples, the pollution data comprises particles per million (PPM) of a given particle in the air, such as Carbon Dioxide, Nitrogen Dioxide, Dust, etc. In some examples, the pollution data comprises Particulate Matter data, such as Particulate Matter 2.5 (PM_{2.5}) data, Particulate Matter 10 (PM₁₀) data, etc.

Pollen data for example comprises information associated with pollen. For example, the pollen data comprises one or more pollen counts, such as values indicative of the quantity of pollen particles per unit volume of air.

Sun radiation data for example comprises information indicative of radiation that the wearer of the wearable device may be exposed to. For example, the sun radiation data comprises UV data associated with the sun, such as a UV index. Sun radiation data may for example comprise solar radiation data.

In some examples, the environment data comprises location data indicative of a stressful location. For example, one or more stressful locations (such as inner-city, high gradient, motorway, etc.) may be pre-determined. The wearable device may be configured to, based on the location data, determine whether the location of the wearable device corresponds to a stressful location.

In one or more example electronic devices, the electronic device 400 is configured to augment the physiological data and/or the physical activity data with one or more data elements of the environment data. Augmenting the physiological data and/or the physical activity data may for example be seen as concatenating, correlating, associating and/or combining the received physiological data and/or the physical activity data with the obtained environment data. The one or more data elements of the environment data are for example associated with a location and/or a timestamp. The electronic device 400 is for example configured to augment the physiological data and/or the physical activity data based on the corresponding location data and/or corresponding timestamp, with weather data, pollution data, and/or sun radiation.

The electronic device 400 may be configured to provide (such as display 12 to a user, such as the user 5) augmented physiological data and/or augmented physical activity data. The augmented physiological data and/or the augmented physical activity data for example comprise one or more data elements of the environment data, and physiological data and/or physical activity data, respectively.

The electronic device 400 may for example be configured to store, such as based on user input, the physiological data, the physical activity data, the augmented physiological data, the augmented physical activity data and/or the environment data.

The electronic device 400 may for example be configured to obtain, such as fetch, such as based on a user input, the physiological data, the physical activity data, the augmented physiological data, the augmented physical activity data and/or the environment data.

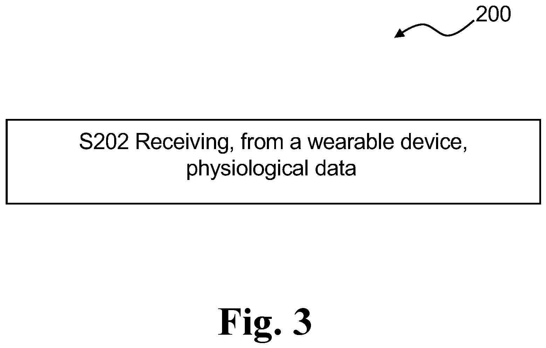

Processor circuitry 402 is optionally configured to perform any of the operations disclosed in Fig. 3 (such as S202). The operations of the electronic device 400 may be embodied in the form of executable logic routines (for example, lines of code, software programs, etc.) that are stored on a non-transitory computer readable medium (for example, memory circuitry 401) and are executed by processor circuitry 402).

Furthermore, the operations of the electronic device 400 may be considered a method that the network node 400 is configured to carry out. Also, while the described functions and operations may be implemented in software, such functionality may also be carried out via dedicated hardware or firmware, or some combination of hardware, firmware and/or software.

Memory circuitry 401 may be one or more of a buffer, a flash memory, a hard drive, a removable media, a volatile memory, a non-volatile memory, a random access memory (RAM), or other suitable device. In a typical arrangement, memory circuitry 401 may include a non-volatile memory for long term data storage and a volatile memory that functions as system memory for processor circuitry 402. Memory circuitry 401 may exchange data with processor circuitry 402 over a data bus. Control lines and an address bus between memory circuitry 401 and processor circuitry 402 also may be present (not shown in Fig. 1). Memory circuitry 401 is considered a non-transitory computer readable medium.

Memory circuitry 401 may be configured to environment data, physiological data, location data, physical activity data, a timestamp and/or a timestamp parameter in a part of the memory.

Fig. 2 shows a flow diagram of an example method 100, performed by a wearable device according to the disclosure, for data collection. The wearable device is the wearable device disclosed herein, such as wearable device 300 of Fig. 1.

The method 100 comprises obtaining S102 physiological data associated with location data. The location data is indicative of a location of the wearable device. The location data has the same timestamp as the physiological data. In some examples, obtaining S102 physiological data associated with location data comprises retrieving and/or receiving physiological data associated with location data. In some examples, obtaining S102 physiological data associated with location data comprises generating physiological data associated with location data.

The method 100 comprises providing S104 the physiological data associated with the location data. For example, providing S104 the physiological data associated with the location data comprises transmitting the physiological data associated with the location data to an external device.

Fig. 3 shows a flow diagram of an example method 200, performed by an electronic device according to the disclosure, for data collection. The electronic device is the electronic device disclosed herein, such as electronic device 400 of Fig. 1.

The method 200 comprises receiving S202, from a wearable device, physiological data. The physiological data is associated with location data indicative of a location of the wearable device. The location data has the same timestamp as the physiological data.

Examples of methods and products (wearable device and electronic device) according to the disclosure are set out in the following items:
Item 1. A wearable device comprising memory circuitry, processor circuitry, an interface, wherein the wearable device is configured to:
   obtain physiological data associated with location data, wherein the location data is indicative of a location of the wearable device, wherein the location data has the same timestamp as the physiological data; and
   provide the physiological data associated with the location data.
Item 2. The wearable device according to item 1, wherein the wearable device is configured to obtain the physiological data by:
   - obtaining the physiological data and the location data from one or more sensors, wherein the one or more sensors comprise one or more sensors internal to the wearable device and/or one or more sensors external to the wearable device.
Item 3. The wearable device according to item 2, wherein the one or more sensors comprise a location sensor, a physiological sensor, and/or a physical activity sensor.
Item 4. The wearable device according to any of items 2-3, wherein the wearable device is configured to obtain the physiological data by:
   generating a timestamp parameter for the physiological data and the location data using the same electronic clock; and
   associating the timestamp parameter with the physiological data and the location data.
Item 5. The wearable device according any of the previous items, wherein the physiological data comprises one or more of: photoplethysmogram data, temperature data, and electrocardiogram data.
Item 6. The wearable device according to any of the previous items, wherein the wearable device is configured to obtain physical activity data associated with the location data, wherein the physical activity data is indicative of a physical activity including movement and/or sleep, wherein the location data has the same timestamp as the physical activity data.
Item 7. The wearable device according to item 6, wherein the wearable device is configured to provide the physical activity data associated with the location data.
Item 8. The wearable device according to any of items 6-7, wherein the physical activity data comprises movement data and/or sleep data, wherein the movement data comprises accelerometer data and/or gyroscope data.
Item 9. The wearable device according to any of the previous items, wherein the interface comprises a wireless interface.
Item 10. The wearable device according to any of the previous items, wherein the location data comprises one or more of: satellite navigation data, cellular positioning data, and beacon positioning data.
Item 11. The wearable device according to any of items 6-10, wherein the wearable device is configured to transmit the physiological data associated with the location data and/or the physical activity data associated with the location data, to an external electronic device.
Item 12. An electronic device comprising memory circuitry, processor circuitry, and an interface, wherein the electronic device is configured to:
   receive, from a wearable device, physiological data, wherein the physiological data is associated with location data indicative of a location of the wearable device, and wherein the location data has the same timestamp as the physiological data.
Item 13. The electronic device according to item 12, wherein the electronic device is configured to receive, physical activity data, wherein the physical activity data is associated with location data indicative of a location of the wearable device, and wherein the location data has the same timestamp as the physical activity data.
Item 14. The electronic device according to any of items 12-13, wherein the electronic device is configured to:
   - receive user input for configuration of the wearable device; and
   - set up one or more configuration parameters associated with the physiological data, location data and/or physical activity data according to the user input.
Item 15. The electronic device according to any of items 12-14, wherein the electronic device is configured to:
   - obtain, based on the physiological data associated with the location data and/or the physical activity data, environment data indicative of conditions at the location and time of the physiological data; and
   - augment the physiological data and/or the physical activity data with one or more data elements of the environment data.
Item 16. The electronic device according to any of items 12-15, wherein the environment data comprises one or more of: weather data, pollution data, pollen data, and sun radiation data.
Item 17. A method, performed by a wearable device, the method comprising:
   obtaining (S102) physiological data associated with location data, wherein the location data is indicative of a location of the wearable device, wherein the location data has the same timestamp as the physiological data; and
   providing (S104) the physiological data associated with the location data.
Item 18. A method, performed by an electronic device, the method comprising:
   receiving (S202), from a wearable device, physiological data, wherein the physiological data is associated with location data indicative of a location of the wearable device, and wherein the location data has the same timestamp as the physiological data.
Item 19. A computer readable storage medium storing one or more programs, the one or more programs comprising instructions, which when executed by a wearable device cause the wearable device to operate according to any of items 1-11.
Item 20. A computer readable storage medium storing one or more programs, the one or more programs comprising instructions, which when executed by an electronic device cause the electronic device to operate according to any of items 12-16.

The use of the terms "first," "second," "third" and "fourth," "primary," "secondary," "tertiary" etc. does not imply any particular order, but are included to identify individual elements. Moreover, the use of the terms "first," "second," "third" and "fourth," "primary," "secondary," "tertiary" etc. does not denote any order or importance, but rather the terms "first," "second," "third" and "fourth," "primary," "secondary", "tertiary" etc. are used to distinguish one element from another.

Note that the words "first," "second," "third" and "fourth," "primary," "secondary," "tertiary" etc. are used here and elsewhere for labelling purposes only and are not intended to denote any specific spatial or temporal ordering. Furthermore, the labelling of a first element does not imply the presence of a second element and vice versa.

It may be appreciated that Figures 1-5 comprise some circuitries or operations which are illustrated with a solid line and some circuitries, components, features, or operations which are illustrated with a dashed line. Circuitries or operations which are comprised in a solid line are circuitries, components, features, or operations which are comprised in the broadest example. Circuitries, components, features, or operations which are comprised in a dashed line are examples which may be comprised in, or a part of, or are further circuitries, components, features, or operations which may be taken in addition to circuitries, components, features, or operations of the solid line examples. It should be appreciated that these operations need not be performed in order presented. Furthermore, it should be appreciated that not all of the operations need to be performed. The example operations may be performed in any order and in any combination. It should be appreciated that these operations need not be performed in order presented. Circuitries, components, features, or operations which are comprised in a dashed line may be considered optional.

Other operations that are not described herein can be incorporated in the example operations. For example, one or more additional operations can be performed before, after, simultaneously, or between any of the described operations.

Certain features discussed above as separate implementations can also be implemented in combination as a single implementation. Conversely, features described as a single implementation can also be implemented in multiple implementations separately or in any suitable sub-combination. Moreover, although features may be described above as acting in certain combinations, one or more features from a claimed combination can, in some cases, be excised from the combination, and the combination may be claimed as any sub-combination or variation of any sub-combination.

It is to be noted that the word "comprising" does not necessarily exclude the presence of other elements or steps than those listed.

It is to be noted that the words "a" or "an" preceding an element do not exclude the presence of a plurality of such elements.

It is to be noted that the term "indicative of" may be seen as "associated with", "related to", "descriptive of," "characterizing", and/or "defining". The terms "indicative of", "associated with", "related to", "descriptive of", "characterizing", and "defining" can be used interchangeably. The term "indicative of" can be seen as indicating a relation. For example, weight data indicative of weight may comprise one or more weight parameters.

It is to be noted that the word "based on" may be seen as "as a function of" and/or "derived from." The terms "based on" and "as a function of" can be used interchangeably. For example, a parameter determined "based on" a data set can be seen as a parameter determined "as a function of" the data set. In other words, the parameter may be an output of one or more functions with the data set as an input.

A function may be characterizing a relation between an input and an output, such as mathematical relation, a database relation, a hardware relation, logical relation, and/or other suitable relations.

It should further be noted that any reference signs do not limit the scope of the claims, that the examples may be implemented at least in part by means of both hardware and software, and that several "means", "units" or "devices" may be represented by the same item of hardware. Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than or equal to 10% of, within less than or equal to 5% of, within less than or equal to 1% of, within less than or equal to 0.1 % of, and within less than or equal to 0.01% of the stated amount. If the stated amount is 0 (such as none, having no), the above recited ranges can be specific ranges, and not within a particular % of the value.

The various example methods, devices, nodes, and systems described herein are described in the general context of method steps or processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program circuitries may include routines, programs, objects, components, data structures, etc. that perform specified tasks or implement specific abstract data types. Computer-executable instructions, associated data structures, and program circuitries represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed disclosure, and it will be made obvious to those skilled in the art that various changes and modifications may be made without departing from the scope of the claimed disclosure. The specification and drawings are, accordingly, to be regarded in an illustrative rather than restrictive sense. The claimed disclosure is intended to cover all alternatives, modifications, and equivalents.

## Claims

1. A wearable device comprising memory circuitry, processor circuitry, an interface, wherein the wearable device is configured to:
obtain physiological data associated with location data, wherein the location data is indicative of a location of the wearable device, wherein the location data has the same timestamp as the physiological data; and
provide the physiological data associated with the location data.

2. The wearable device according to claim 1, wherein the wearable device is configured to obtain the physiological data by:
obtaining the physiological data and the location data from one or more sensors, wherein the one or more sensors comprise one or more sensors internal to the wearable device and/or one or more sensors external to the wearable device.

3. The wearable device according to claim 2, wherein the one or more sensors comprise a location sensor, a physiological sensor, and/or a physical activity sensor.

4. The wearable device according to any of the previous claims, wherein the wearable device is configured to obtain the physiological data by:
generating a timestamp parameter for the physiological data and the location data using the same electronic clock; and
associating the timestamp parameter with the physiological data and the location data.

5. The wearable device according to any of the previous claims, wherein the physiological data comprises one or more of: photoplethysmogram data, temperature data, and electrocardiogram data.

6. The wearable device according to any of the previous claims, wherein the wearable device is configured to:
obtain physical activity data associated with the location data, wherein the physical activity data is indicative of a physical activity including movement and/or sleep, wherein the location data has the same timestamp as the physical activity data; and
provide the physical activity data associated with the location data.

7. The wearable device according to claim 6, wherein the physical activity data comprises movement data and/or sleep data, wherein the movement data comprises accelerometer data and/or gyroscope data.

8. The wearable device according to any of the previous claims, wherein the interface comprises a wireless interface.

9. The wearable device according to any of the previous claims, wherein the location data comprises one or more of: satellite navigation data, cellular positioning data, and beacon positioning data.

10. The wearable device according to any of claims 6-9, wherein the wearable device is configured to transmit the physiological data associated with the location data and/or the physical activity data associated with the location data, to an external electronic device.

11. An electronic device comprising memory circuitry, processor circuitry, and an interface, wherein the electronic device is configured to:
receive, from a wearable device, physiological data, wherein the physiological data is associated with location data indicative of a location of the wearable device, and wherein the location data has the same timestamp as the physiological data.

12. The electronic device according to claim 11, wherein the electronic device is configured to receive, physical activity data, wherein the physical activity data is associated with location data indicative of a location of the wearable device, and wherein the location data has the same timestamp as the physical activity data.

13. The electronic device according to any of claims 11-12, wherein the electronic device is configured to:
receive user input for configuration of the wearable device; and
set up one or more configuration parameters associated with the physiological data, location data and/or physical activity data according to the user input.

14. A method, performed by a wearable device, the method comprising:
obtaining (S102) physiological data associated with location data, wherein the location data is indicative of a location of the wearable device, wherein the location data has the same timestamp as the physiological data; and
providing (S104) the physiological data associated with the location data.

15. A method, performed by an electronic device, the method comprising:
receiving (S202), from a wearable device, physiological data, wherein the physiological data is associated with location data indicative of a location of the wearable device, and wherein the location data has the same timestamp as the physiological data.
